# EUROPEAN PATENT SPECIFICATION

(11) **EP 4 394 387 B1**
(45) Date of publication and mention of the grant of the patent: **15.04.2026**
(21) Application number: 23214241.4
(22) Date of filing: 05.12.2023
(51) Int. Cl.: G01N 35/00

(54) **REAGENT PREPARATION APPARATUS AND NUCLEIC ACID TESTING INTEGRATED MACHINE WITH REAGENT PREPARATION APPARATUS**
REAGENZHERSTELLUNGSVORRICHTUNG UND INTEGRIERTE NUKLEINSÄURETESTMASCHINE MIT REAGENZHERSTELLUNGSVORRICHTUNG
APPAREIL DE PRÉPARATION DE RÉACTIF ET MACHINE INTÉGRÉE DE TEST D'ACIDE NUCLÉIQUE AVEC APPAREIL DE PRÉPARATION DE RÉACTIF

(30) Priority: 31.12.2022 CN 202211738460
(43) Date of publication of application: 03.07.2024
(73) Proprietor: Shenzhen New Industries Biomedical Engineering Co., Ltd., Shenzhen, Guangdong 518122 (CN)
(72) Inventor: WANG, Yixian, Shenzhen, 518122 (CN); ZHENG, Xiaolin, Shenzhen, 518122 (CN); HE, Guoyao, Shenzhen, 518122 (CN); LIU, Zhenhua, Shenzhen, 518122 (CN); XIONG, Yingchao, Shenzhen, 518122 (CN); ZHANG, Tingting, Shenzhen, 518122 (CN); CHEN, Muxiao, Shenzhen, 518122 (CN); MA, Juanjuan, Shenzhen, 518122 (CN); SHENG, Xueqing, Shenzhen, 518122 (CN)
(74) Representative: Studio Torta S.p.A.

(56) References cited:
- CN-U- 214 167 961
- CN-U- 218 146 703
- US-A1- 2019 107 547

## Description

### Technical Field

The invention relates to a technical field of biological detection, in particular to a reagent preparation apparatus and a nucleic acid testing integrated machine with the reagent preparation apparatus.

### Background

Nucleic acid testing usually needs to extract nucleic acid from a subject sample, then perform an amplification reaction, and further analyze a situation of the nucleic acid in a subject body by detecting an amplification result. This testing process involves the addition of a variety of extraction reagents and amplification reagents, as well as the loading and use of a variety of vessel consumables.

In the related art, the preparation of the extraction reagents and the amplification reagents is often allocated to a plurality of instrument modules, which easily leads to the problem that a nucleic acid testing instrument is too large. However, the space in hospital laboratories is limited, and a method of arranging a plurality of modules separately for reagent addition is difficult to meet the requirements for miniaturization of the nucleic acid testing instrument on the market.

Examples of a reagent preparation apparatus and a nucleic acid testing integrated machine with the reagent preparation apparatus according to the prior art are known from CN214167961 U and CN218146703U.

### Summary

The invention provides a reagent preparation apparatus and a nucleic acid testing integrated machine with the reagent preparation apparatus, so as to solve the problem that a nucleic acid testing instrument in the related technology cannot meet the requirements for miniaturization.

The invention is set out in the appended set of claims

According to one aspect of the invention, a reagent preparation apparatus is provided. The reagent preparation apparatus includes: a housing, the housing being provided with an extraction reagent filling position and an amplification reagent filling position; a reagent storage portion, arranged in the housing, the reagent storage portion including an extraction reagent storage portion and an amplification reagent storage portion; and a filling portion, movably arranged in the housing, the filling portion being able to suck the extraction reagent from the extraction reagent storage portion and move to above the extraction reagent filling position to fill the extraction reagent into a first vessel on the extraction reagent filling position, and the filling portion being able to suck the amplification reagent from the amplification reagent storage portion and move to above the amplification reagent filling position to fill the amplification reagent into a second vessel on the amplification reagent filling position.

The housing is provided with a first platform and a second platform located below the first platform, the first platform and the second platform are parallel to a horizontal plane, the extraction reagent storage portion, the amplification reagent storage portion, the extraction reagent filling position, and the amplification reagent filling position are arranged on the second platform, and the filling portion is arranged on the first platform.

In some embodiments, the reagent preparation apparatus further includes a first vessel loading mechanism configured to load the first vessel and a grabbing piece. The first vessel loading mechanism includes a first vessel loading bin and a first vessel transfer bin. The housing is further provided with a third platform located below the second platform and parallel to the horizontal plane. The first vessel loading bin is movably arranged on the third platform and able to move to the outside of the housing. The first vessel transfer bin is fixedly arranged on the third platform. The top of the first vessel transfer bin is provided with a first opening, and the first opening extends to above the second platform. The grabbing piece is movably arranged on the first platform, and the grabbing piece is able to grab the first vessel through the first opening and move to the extraction reagent filling position.

In some embodiments, the first vessel loading bin and the first vessel transfer bin extend in a Z-axis direction and are arranged at an interval in an X-axis direction, the housing is provided with a first side opening, and the first vessel loading bin is able to move to the outside of the housing through the first side opening.

In some embodiments, the reagent preparation apparatus further includes a second vessel loading mechanism configured to load the second vessel. The second vessel loading mechanism includes a second vessel loading bin. The second vessel loading bin is movably arranged in the housing and able to move to the outside of the housing, the second vessel loading bin is provided with a second opening, and the gripping piece is able to grab the second vessel through the second opening.

In some embodiments, the second vessel loading mechanism further includes a second vessel storage bin. The second vessel storage bin is fixedly arranged on the second platform and extends in the Z-axis direction, and the second vessel loading bin is movably arranged on the second platform in the X-axis direction and extends in the Z-axis direction. The housing is further provided with a second side opening located above the first side opening. The second vessel loading bin is able to move to the outside of the housing through the second side opening. The top of the second vessel storage bin is provided with a third opening. The grabbing piece is able to transfer the second vessel from the second vessel loading bin to the second vessel storage bin, and the grabbing piece is able to grab the second vessel through the third opening and move to the amplification reagent filling position.

The extraction reagent storage portion includes a first reagent storage portion and a second reagent storage portion. In some embodiments, the first reagent storage portion is arranged on the second platform, the second reagent storage portion is arranged outside of the housing, the first reagent storage portion and the second vessel loading bin are arranged at an interval in the X-axis direction, and the first reagent storage portion is able to move to the outside of the housing with the second vessel loading bin.

In some embodiments, the second platform is further provided with a premixing position configured to place the first vessel, and the grabbing piece is able to grab the first vessel through the first opening and move to the premixing position.

The filling portion further includes a filling assembly and a reagent arm assembly. The filling assembly includes a moving frame and a liquid injection needle. The moving frame is movably arranged in the housing in a Y-axis direction, the liquid injection needle is movably arranged on the moving frame in the Z-axis direction, and the liquid injection needle communicates with the second reagent storage portion to fill a second reagent into the first vessel. The reagent arm assembly includes a reagent arm and a pipetting needle. The reagent arm is movably arranged on the first platform in the X-axis direction and the Y-axis direction, the pipetting needle is movably arranged on the reagent arm in the Z-axis direction, the pipetting needle is able to move to above the first reagent storage portion to suck a first reagent so as to fill the first reagent into the first vessel, and the pipetting needle is able to move to above the amplification reagent storage portion to suck the amplification reagent and move to above the amplification reagent filling position to fill the amplification reagent into the second vessel.

In some embodiments, the first platform is provided with a first guide rail and a second guide rail, the first guide rail extends in the Y-axis direction, the second guide rail extends in the X-axis direction, the first guide rail is movably arranged below the second guide rail in an extension direction of the second guide rail, and the reagent arm is movably arranged on the first guide rail in an extension direction of the first guide rail.

In some embodiments, the first platform is further provided with a third guide rail extending in the Y-axis direction, and the third guide rail is movably arranged below the second guide rail in the extension direction of the second guide rail. The grabbing piece includes a grabbing frame and a gripper. The grabbing frame is movably arranged on the third guide rail in an extension direction of the third guide rail, and the gripper is movably arranged on the grabbing frame in the Z-axis direction.

In some embodiments, the reagent preparation apparatus further includes a consumable storage bin arranged on the second platform and configured to carry a magnetic rod sleeve and a pipette tip. The pipetting needle is able to move to above the consumable storage bin to load the magnetic rod sleeve and the pipette tip, and the consumable storage bin and the first reagent storage portion extend in the X-axis direction and are arranged at an interval in the Y-axis direction.

In some embodiments, the reagent preparation apparatus further includes a first vessel transfer assembly arranged on the second platform. The first vessel transfer assembly includes a transfer frame, a sliding plate, and a sliding plate driving piece. The extraction reagent filling position is arranged on an upper surface of the sliding plate, the transfer frame is arranged on the second platform and extends in the X-axis direction, the sliding plate driving piece is arranged on the transfer frame, and the sliding plate driving piece is in drive connection with the sliding plate, so that the sliding plate moves in an extension direction of the transfer frame.

In some embodiments, an upper surface of the extraction reagent filling position is lower than an upper surface of the amplification reagent filling position.

According to another aspect of the invention, a nucleic acid testing integrated machine is provided. The nucleic acid testing integrated machine includes a reagent preparation apparatus, a to-be-tested object extraction apparatus, and an amplification apparatus. The to-be-tested object extraction apparatus is configured to extract an analyte from a sample and mix the analyte with the amplification reagent to form a determination mixture, the amplification apparatus is configured to perform analyte detection determination on the determination mixture to analyze the sample, and the reagent preparation apparatus is the above reagent preparation apparatus.

By applying the technical solutions of the invention, the reagent preparation apparatus includes the housing, the reagent storage portion, and the filling portion, when preparing the extraction reagent and the amplification reagent, the first vessel is placed on the extraction reagent filling position, the second vessel is placed on the amplification reagent filling position, the filling portion which is movably arranged in the housing moves to above the extraction reagent storage portion to suck the extraction reagent and release the extraction reagent into the first vessel, and the filling portion moves to above the amplification reagent storage portion to suck the amplification reagent and release the amplification reagent into the second vessel. Because the preparation of the amplification reagent and the extraction reagent is completed by using the same filling portion arranged in the housing, the miniaturization of the reagent preparation apparatus is facilitated.

### Brief Description of the Drawings

The accompanying drawings of the specification, which constitute a part of the invention, are intended to provide a further understanding of the invention, and the exemplary embodiments of the invention and the description thereof are intended to explain the invention and do not constitute an undue limitation on the invention. In the accompanying drawings:
Fig. 1 shows a schematic structural diagram of a reagent preparation apparatus according to an embodiment of the invention.
Fig. 2 shows a schematic structural diagram of a housing, a first vessel loading mechanism, and a second vessel loading mechanism of a reagent preparation apparatus according to an embodiment of the invention.
Fig. 3 shows a schematic structural diagram of a first vessel loading mechanism of a reagent preparation apparatus according to an embodiment of the invention.
Fig. 4 shows a schematic structural diagram of an extraction reagent storage portion and a second vessel loading bin of a reagent preparation apparatus according to an embodiment of the invention.
Fig. 5 shows a schematic structural diagram of a second vessel storage bin of a reagent preparation apparatus according to an embodiment of the invention.
Fig. 6 shows a schematic structural diagram of a reagent arm and a grabbing piece of a reagent preparation apparatus according to an embodiment of the invention.
Fig. 7 shows a schematic structural diagram of a reagent arm of a reagent preparation apparatus according to an embodiment of the invention.
Fig. 8 shows a schematic structural diagram of a gripper of a reagent preparation apparatus according to an embodiment of the invention.
Fig. 9 shows a schematic structural diagram of a moving frame of a reagent preparation apparatus according to an embodiment of the invention.
Fig. 10 shows a schematic structural diagram of a first vessel transfer assembly of a reagent preparation apparatus according to an embodiment of the invention.

Herein, the above accompanying drawings include the following reference signs:
10: Housing; 11: Extraction reagent filling position; 12: Amplification reagent filling position; 13: First platform; 14: Second platform; 141: Premixing position; 15: First guide rail; 16: Second guide rail; 17: Third guide rail; 18: Third platform;
20: Reagent storage portion; 21: Extraction reagent storage portion; 211: First reagent storage portion; 22: Amplification reagent storage portion;
30: Filling portion; 31: Moving frame; 32: Liquid injection needle; 33: Reagent arm; 34: Pipetting needle;
40: First vessel loading mechanism; 41: First vessel loading bin; 42: First vessel transfer bin; 421: First opening;
50: Grabbing piece; 51: Grabbing frame; 52: Gripper;
60: Second vessel loading mechanism; 61: Second vessel loading bin; 611: Second opening; 62: Second vessel storage bin; 621: Third opening;
70: Consumable storage bin;
80: First vessel transfer assembly; 81: Transfer frame; 82: Sliding plate.

### Detailed Description of the Embodiments

The technical solutions in the embodiments of the invention will be clearly and completely described in conjunction with the accompanying drawings in the embodiments of the invention. It is apparent that the described embodiments are only a part of the embodiments of the invention, and not all of them. The following description of at least one exemplary embodiment is only illustrative, and in no way serves as any limitation on the invention or application or use thereof. All other embodiments obtained by those of ordinary skill in the art based on the embodiments of the invention without creative efforts are within the scope of protection of the invention.

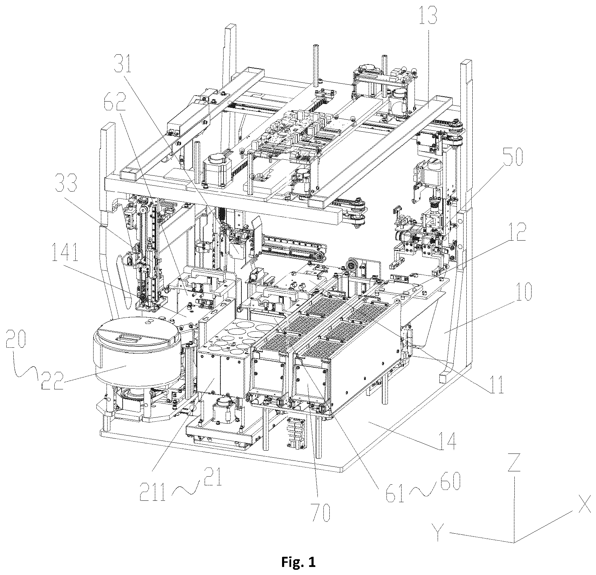

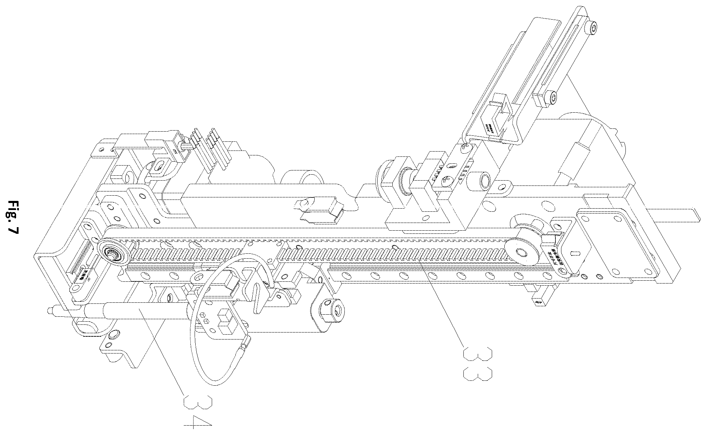

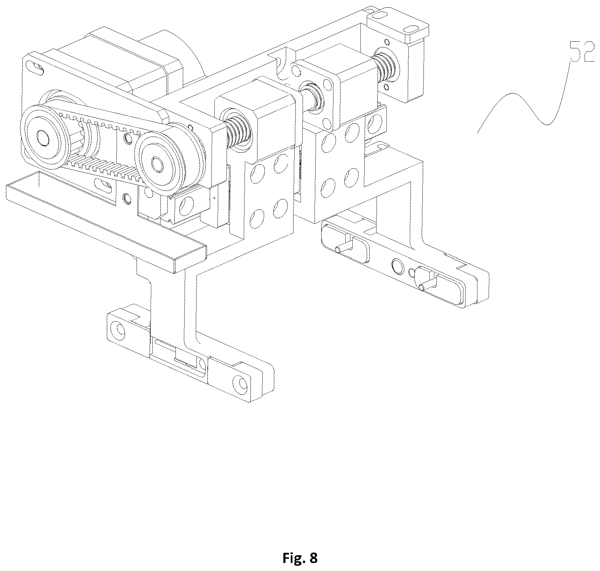

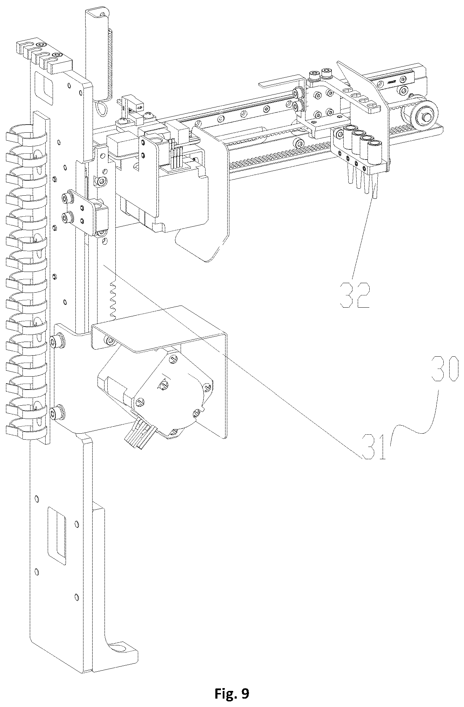

As shown in Fig. 1 to Fig. 10, the embodiments of the invention provide a reagent preparation apparatus. The reagent preparation apparatus includes: a housing 10, a reagent storage portion 20, and a filling portion 30. The housing 10 is provided with an extraction reagent filling position 11 and an amplification reagent filling position 12. The reagent storage portion 20 is arranged in the housing 10, and the reagent storage portion 20 includes an extraction reagent storage portion 21 and an amplification reagent storage portion 22. The filling portion 30 is movably arranged in the housing 10, the filling portion 30 is able to suck an extraction reagent from the extraction reagent storage portion 21 and move to above the extraction reagent filling position 11 to fill the extraction reagent into a first vessel on the extraction reagent filling position 11, and the filling portion 30 is able to suck an amplification reagent from the amplification reagent storage portion 22 and move to above the amplification reagent filling position 12 to fill the amplification reagent into a second vessel on the amplification reagent filling position 12.

By applying the technical solutions of the invention, the reagent preparation apparatus includes the housing 10, the reagent storage portion 20, and the filling portion 30, when preparing the extraction reagent and the amplification reagent, the first vessel is placed on the extraction reagent filling position 11, the second vessel is placed on the amplification reagent filling position 12, the filling portion 30 which is movably arranged in the housing 10 moves to above the extraction reagent storage portion 21 to suck the extraction reagent and release the extraction reagent into the first vessel, and the filling portion 30 moves to above the amplification reagent storage portion 22 to suck the amplification reagent and release the amplification reagent into the second vessel. Because the preparation of the amplification reagent and the extraction reagent is completed by using the same filling portion arranged in the housing, the miniaturization of the reagent preparation apparatus is facilitated.

Moreover, because the preparation of the extraction reagent and the amplification reagent is placed in a separate reagent preparation apparatus, a sample cannot pollute the extraction reagent and the amplification reagent, so that a testing result is more accurate.

The first vessel and the second vessel are orifice plates.

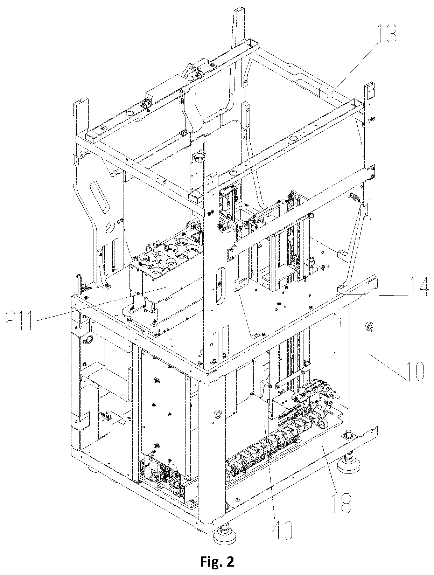

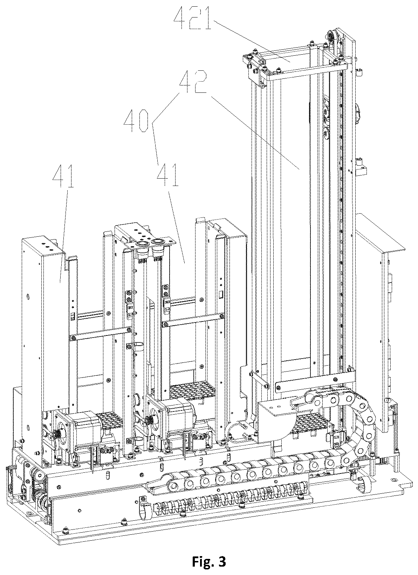

As shown in Fig. 2 and Fig. 3, the housing 10 is provided with a first platform 13 and a second platform 14 located below the first platform 13, the first platform 13 and the second platform 14 are parallel to a horizontal plane, the extraction reagent storage portion 21, the amplification reagent storage portion 22, the extraction reagent filling position 11, and the amplification reagent filling position 12 are arranged on the second platform 14, and the filling portion 30 is arranged on the first platform 13. Through the adoption of the above structure, the first platform 13 and the second platform 14 are arranged at an interval along a Z-axis, so that the space of the reagent preparation apparatus in a Z-axis direction is fully utilized, thereby facilitating the miniaturization of the apparatus.

As shown in Fig. 1, Fig. 3, and Fig. 6, the reagent preparation apparatus further includes a first vessel loading mechanism 40 configured to load the first vessel and a grabbing piece 50. The first vessel loading mechanism 40 includes a first vessel loading bin 41 and a first vessel transfer bin 42. The housing 10 is further provided with a third platform 18 located below the second platform 14 and parallel to the horizontal plane. The first vessel loading bin 41 is movably arranged on the third platform 18 and is able to move to the outside of the housing 10. The first vessel transfer bin 42 is fixedly arranged on the third platform 18. The top of the first vessel transfer bin 42 is provided with a first opening 421, and the first opening 421 extends to above the second platform 14. The grabbing piece 50 is movably arranged on the first platform 13, and the grabbing piece 50 is able to grab the first vessel through the first opening 421 and move to the extraction reagent filling position 11. By using the first vessel loading mechanism 40, a user is able to place the first vessel to the outside of the housing, and transfer it to the inside of the housing. By using the grabbing piece 50, the first vessel is transferred from the first vessel transfer bin 42 to the extraction reagent filling position 11, which has the advantage of compact structure. When the grabbing piece 50 grabs the first vessel in the first vessel transfer bin 42, it does not affect the user to place the first vessel in the first vessel loading bin 41, so as to realize the continuous loading of the first vessels.

The first vessel loading bin 41 is provided with a first pallet movable along the Z-axis, the first vessel transfer bin 42 is provided with a second pallet movable along the Z-axis, the first vessel is placed on the first pallet and moves along the Z-axis with the first pallet in the first vessel loading bin 41, and the first vessel is placed on the second pallet and moves along the Z-axis with the second pallet in the first vessel transfer bin 42.

Specifically, the top of the first vessel loading bin 41 is provided with a loading opening to facilitate the user to place the first vessel in the first vessel loading bin 41 through the loading opening.

In the embodiment, the first vessel loading mechanism 40 further includes a horizontal transfer assembly. The horizontal transfer assembly includes two rotating members and two conveyor belts. The two rotating members are respectively arranged below the first vessel loading bin 41 and the first vessel transfer bin 42 and rotate along own axes. Each conveyor belt rotationally sleeves the two rotating members. An interval is provided between the two conveyor belts. The bottom of the first vessel loading bin 41 is provided with a first avoidance opening, the bottom of the first vessel transfer bin 42 is provided with a second avoidance opening, the first pallet is able to move to below the interval through the first avoidance opening, so that the first vessel is located on the two conveyor belts, and the second pallet is able to move to below the interval through the second avoidance opening, so that the second pallet is able to support the pallet on the two conveyor belts. Through the adoption of the above structure, the first vessel in the first vessel loading bin 41 is transferred to below the first vessel transfer bin 42 by using the horizontal transfer assembly, and the second pallet is used to move the first vessel from the bottom of the first vessel transfer bin 42 to the top of the first vessel transfer bin 42, so that the grabbing piece 50 is able to grab the first vessel.

In the embodiment, a lower part of the first vessel loading bin 41 is provided with a drawer type pull-out structure, so that the first vessel loading bin 41 is able to move from the inside of the housing 10 to the outside of the housing 10. The reagent preparation apparatus includes two first vessel loading bins 41 arranged at an interval in an X-axis direction, the two first vessel loading bins 41 being pulled to the outside of the housing 10.

Specifically, the first vessel loading mechanism 40 further includes an overturning piece which is overturned on one side of the first vessel loading bin 41. The overturning piece is provided with a supporting position for supporting the first vessel and an avoidance position for avoiding the first vessel. The first vessel is supported by using the overturning piece, when the first vessel is transferred to the conveyor belt with the first pallet, the first pallet and the first vessel located on the first pallet are avoided by using the overturning piece in the avoidance position, and one first vessel above another first vessel on the first pallet is supported by using the overturning piece in the supporting position, so as to separate stacked extraction plates.

In the embodiment, the overturning piece is driven by a motor to realize the overturning.

As shown in Fig. 2 and Fig. 3, the first vessel loading bin 41 and the first vessel transfer bin 42 extend in the Z-axis direction and are arranged at an interval in the X-axis direction, the housing 10 is provided with a first side opening, and the first vessel loading bin 41 is able to move to the outside of the housing 10 through the first side opening. Through the adoption of the above arrangement, the space of the reagent preparation apparatus in the Z-axis direction is fully utilized, which facilitates the miniaturization of the apparatus.

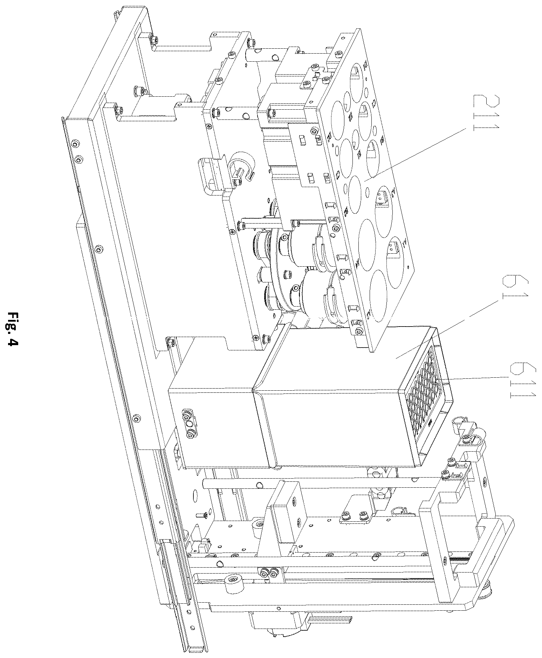

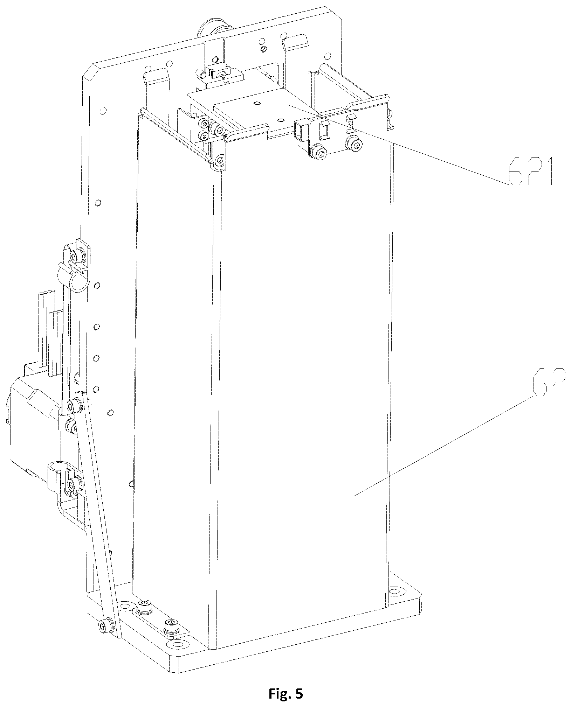

As shown in Fig. 4 and Fig. 5, the reagent preparation apparatus further includes a second vessel loading mechanism 60 configured to load the second vessel. The second vessel loading mechanism 60 includes a second vessel loading bin 61. The second vessel loading bin 61 is movably arranged in the housing 10 and is able to move to the outside of the housing 10, the second vessel loading bin 61 is provided with a second opening 611, and the gripping piece 50 is able to grab the second vessel through the second opening 611. By using the second vessel loading mechanism 60, the user is able to place the second vessel to the outside of the housing, and transfer it to the inside of the housing 10.

As shown in Fig. 4 and 5, the second vessel loading mechanism 60 further includes a second vessel storage bin 62. The second vessel storage bin 62 is fixedly arranged on the second platform 14 and extends in the Z-axis direction, and the second vessel loading bin 61 is movably arranged on the second platform 14 in the X-axis direction and extends in the Z-axis direction. The housing 10 is further provided with a second side opening located above the first side opening. The second vessel loading bin 61 is able to move to the outside of the housing 10 through the second side opening. The top of the second vessel storage bin 62 is provided with a third opening 621. The grabbing piece 50 is able to transfer the second vessel from the second vessel loading bin 61 to the second vessel storage bin 62, and the grabbing piece 50 is able to grab the second vessel through the third opening 621 and move to the amplification reagent filling position 12. The second vessels in the second vessel transfer bin 61 are transferred to the second vessel storage bin 62 in sequence by using the grabbing piece 50, and when the grabbing piece 50 grabs the second vessel in the second vessel storage bin 62, it does not affect the user to place the second vessel in the second vessel loading bin 61, so as to realize the continuous loading of the second vessels.

The second vessel loading bin 61 is provided with a third pallet movable in the Z-axis direction, the second vessel transfer bin 62 is provided with a fourth pallet moving in the Z-axis direction, the second vessel is placed on the third pallet and moves along with the third pallet in the second vessel loading bin 61, and the second vessel is placed on the fourth pallet and moves along with the fourth pallet in the second vessel transfer bin 62 in the Z-axis direction.

Specifically, the grabbing piece 50 grabs the second vessel in the second vessel loading bin 61 and moves to above the second vessel storage bin 62, the fourth pallet moves to the third opening 621 at the top of the second vessel storage bin 62 in the extension direction of the second vessel storage bin 62, and the grabbing piece 50 releases the second vessel onto the fourth pallet. After the No.1 second vessel is transferred, the No.2 second vessel located below the No.1 second vessel is grabbed from the second vessel loading bin 61 by using the grabbing piece 50, and the fourth pallet moves down a certain distance in the extension direction of the second vessel storage bin 62, so that the No.2 second vessel is placed on the No,1 second vessel. By analogy, all the second vessels in the second vessel loading bin 61 are transferred to the second vessel storage bin 62. That is, the second vessel at the lower part of the second vessel loading bin 61 is transferred to the upper part of the second vessel loading bin 62, and the second vessel at the upper part of the second vessel loading bin 61 is transferred to the lower part of the second vessel storage bin 62, so that the second vessel first placed in the second vessel loading bin 61 is preferentially transferred to the second vessel storage bin 62 for use.

Of course, the second vessel is also grabbed by using the grabbing piece 50 through the second opening 611, and the second vessel is directly transferred to the amplification reagent filling position 12 for the preparation of the amplification reagent.

As shown in Fig. 4, the extraction reagent storage portion includes a first reagent storage portion 211 and a second reagent storage portion. The first reagent storage portion 211 is arranged on the second platform 14, the second reagent storage portion is arranged outside the housing 10, the first reagent storage portion 211 and the second vessel loading bin 61 are arranged at an interval in the X-axis direction, and the first reagent storage portion 211 is able to move to the outside of the housing 10 with the second vessel loading bin 61. Through the adoption of the above structure, the first reagent storage portion 211 and the second reagent storage portion which are arranged in the X-axis direction of the apparatus are fully utilized, and the first reagent storage portion 211 and the second vessel loading bin 61 are arranged on the same base movable in the X-axis direction, and move to the outside of the housing 10 together to facilitate the miniaturization of the apparatus.

Specifically, the first reagent storage portion 211 includes a magnetic bead bin body, an elution solution bin body, a rotary plate, and a bin body driving piece. The magnetic bead bin body and the elution solution bin body are arranged in the housing 10, the rotary plate is rotationally arranged in the magnetic bead bin body, and the bin body driving piece is in drive connection with the rotary plate to enable the rotary plate to rotate along its own axis. The bin body driving piece is able to drive the rotary plate to rotate, so that magnetic beads in the magnetic bead bin body are always in a mixed state.

It is to be noted that because the magnetic beads are prone to precipitation, the magnetic beads are kept in a mixed state by arranging the rotary magnetic bead bin body. Furthermore, in order to prevent the precipitation of the magnetic beads in the transfer process, a "one suction one discharge" filling mode is adopted only, that is, after sucking magnetic bead liquid from the magnetic bead bin body, the liquid is discharged in a reagent tank immediately, and a "one suction multi-discharge" filling mode is not adopted. However, in the process of elution solution suction and filling, after the reagent is sucked from the elution solution bin body once, the first vessel is filled many times.

As shown in Fig. 1, the second platform 14 is further provided with a premixing position 141 configured to place the first vessel, and the grabbing piece 50 is able to grab the first vessel through the first opening 421 and move to the premixing position 141. By using the premixing position 141, the amplification reagent is extracted and premixed in the first vessel, so that the amplification reagent is more uniformly mixed.

It is to be noted that there are two kinds of amplification reagents, and the two kinds of amplification reagents are stored in the amplification reagent storage portion, and are respectively placed in a first amplification reagent bottle and a second amplification reagent bottle. The first amplification reagent bottle is provided with a first amplification reagent, the second amplification reagent bottle is provided with a second amplification reagent, and mixed liquor formed after the first amplification reagent and the second amplification reagent are mixed is configured for the amplification of a sample.

In the embodiment, the first vessel not only realizes the preparation of the extraction reagent, but also is used as a premixing plate to realize the mixing of the first amplification reagent and the second amplification reagent.

Specifically, when the amplification reagent is prepared, a pipetting needle 34 moves to above the amplification reagent storage portion 22 to respectively suck the first amplification reagent in the first amplification reagent bottle and the second amplification reagent in the second amplification reagent bottle, and the pipetting needle 34 moves to above the first vessel to fill the first amplification reagent and the second amplification reagent into the first vessel to be mixed into the mixed liquor, and then the pipetting needle 34 sucks the mixed liquor and moves to the filling position to fill the mixed liquid into the second vessel, thereby completing the preparation of the amplification reagent, which has the advantage of high efficiency compared with a method of preparing the amplification reagent manually.

The amplification reagent storage portion 22 includes an amplification reagent bin, a base plate frame, and a base plate. The base plate frame is arranged on the second platform 14, the amplification reagent bin is arranged on the base plate frame, the base plate is rotationally arranged in the amplification reagent bin along its own axis, and the base plate is provided with a plurality of first placement position configured to place the first amplification reagent bottle and a plurality of second placement positions configured to place the amplification reagent bottle. The amplification reagent storage portion 22 adopting the above structure uses the rotation of the base plate to enable a pipette tip to correspond to the first amplification reagent bottle and the second amplification reagent bottle to suck the first amplification reagent and the second amplification reagent, which has the advantages of being easy to operate.

The second platform 14 is further provided with a cover body caching position, the second vessel includes an orifice plate and a cover body covering the upper surface of the orifice plate, and the grabbing piece 50 is able to grab the cover body and move to the cover body caching position. The cover body is placed by using the cover body caching position, which facilitates the filling of the amplification reagent.

In the embodiment, the amplification reagent is filled into the reagent tank, and compression bonded encapsulation is performed on the prepared amplification reagent by using the cover body. Before the preparation of the extraction reagent of the second vessel, the grabbing piece 50 is needed to use to move the cover body to the cover body caching position.

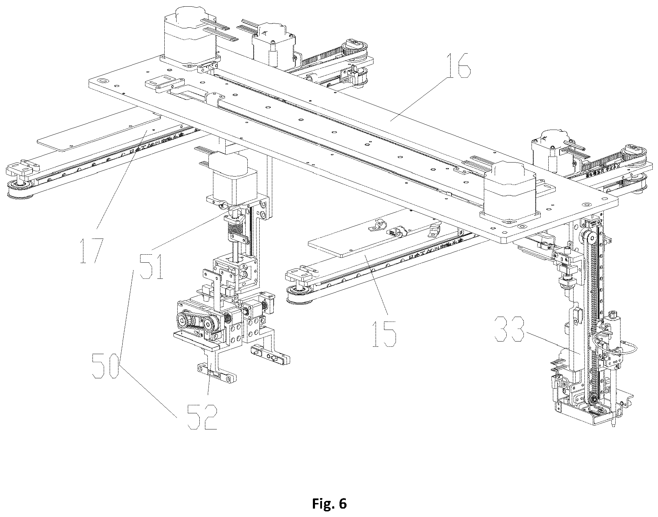

As shown in Fig. 6, the filling portion 30 includes a filling assembly. The filling assembly includes a moving frame 31 and a liquid injection needle 32. The moving frame 31 is movably arranged in the housing 10 in a Y-axis direction, the liquid injection needle 32 is movably arranged on the moving frame 31 in the Z-axis direction, and the liquid injection needle 32 communicates with the second reagent storage portion to fill a second reagent into the first vessel. Through the adoption of the above structure, the two-axis movement of the liquid injection needle 32 in a vertical plane is realized by using the moving frame 31, and then the second reagent is filled into the first vessel.

In the embodiment, the second reagent includes lysate, first washing liquid, second washing liquid, and third washing liquid for the removal of impurities such as cellular components other than an analyte in the sample. The moving frame 31 is provided with four liquid injection needles 32, respectively capable of filling the lysate, the first washing liquid, the second washing liquid, and the third washing liquid into the corresponding reagent tank in the first vessel.

The filling portion 30 further includes a reagent arm assembly. The reagent arm assembly includes a reagent arm 33 and a pipetting needle 34. The reagent arm 33 is movably arranged on the first platform 13 in the X-axis direction and the Y-axis direction, the pipetting needle 34 is movably arranged on the reagent arm 33 in the Z-axis direction, the pipetting needle 34 is able to move to above the first reagent storage portion 211 to suck a first reagent so as to fill the first reagent into the first vessel, the pipetting needle 34 is able to move to above the amplification reagent storage portion 22 to suck the amplification reagent and moves to above the amplification reagent filling position 12 to fill the amplification reagent into the second vessel. The three-axis movement of the pipetting needle 34 is realized by using the reagent arm 33, so that the pipetting needle 34 is able to fill the first reagent and the amplification reagent respectively.

In the embodiment, the first reagent includes the elution solution and the magnetic beads. When extracting the nucleic acid of the same kind, the demand for the lysate, the first washing liquid, the second washing liquid, and the third washing liquid in different reagent tanks is about 500 µL to 600 µL, the demand for the magnetic beads is about 80 µL, and the demand for the elution solution is about 150 µL. For the second reagent with large demand, the liquid injection needle 32 is used to fill directly. For the first reagent with small demand, a control element is arranged in the reagent arm 33 to precisely control the suction amount of the reagent. Through the adoption of the above classification and extraction method, the filling efficiency of the extraction reagent is greatly improved.

In some other embodiments, the filling portion 30 may further include only the reagent arm 33 and the pipetting needle 34. The reagent arm 33 is movably arranged in the housing 10 in the X-axis direction and the Y-axis direction, the pipetting needle 34 is movably arranged on the reagent arm 33 in the Z-axis direction, the pipetting needle 34 is able to move to above the first reagent storage portion 21 to suck the extraction reagent and move to above the extraction agent filling position 11 to fill the extraction reagent to the extraction plate, and the pipetting needle 34 is able to move to above the amplification reagent storage portion 22 to suck the amplification reagent and move to above the amplification reagent filling position 12 to fill the amplification reagent to an amplification plate. The three-axis movement of the pipetting needle 34 is realized by using the reagent arm 33, so that the pipetting needle 34 is able to fill the extraction reagent and the amplification reagent respectively.

As shown in Fig. 6, the housing 10 is provided with a first guide rail 15 and a second guide rail 16, the first guide rail 15 extends in the Y-axis direction, the second guide rail 16 extends in the X-axis direction, the first guide rail 15 is movably arranged below the second guide rail 16 in an extension direction of the second guide rail 16, and the reagent arm 33 is movably arranged on the first guide rail 15 in an extension direction of the first guide rail 15. Through the adoption of the first guide rail 15 and the second guide rail 16, the movement of the reagent arm 33 in a plane parallel to the horizontal plane is realized, so as to realize the three-axis movement of the pipetting needle 34, which has the advantage of simple structure.

As shown in Fig. 6, the housing 10 is further provided with a third guide rail 17 extending in the Y-axis direction, and the third guide rail 17 is movably arranged below the second guide rail 16 in the extension direction of the second guide rail 16. The grabbing piece 50 includes a grabbing frame 51 and a gripper 51. The grabbing frame 51 is movably arranged on the third guide rail 17 in an extension direction of the third guide rail 17, and the gripper 52 is movably arranged on the grabbing frame 51 in the Z-axis direction. The three-axis movement of the gripper 52 is realized by using the third guide rail 17, which has the advantage of simple structure. Moreover, because the first guide rail 15 and the third guide rail 17 are arranged on the same second guide rail 16, the structure is more compact.

As shown in Fig. 1, the reagent preparation apparatus further includes a consumable storage bin 70 arranged on the second platform 14 and configured to carry a magnetic rod sleeve and the pipette tip. The pipetting needle 34 is able to move to above the consumable storage bin 70 to load the magnetic rod sleeve and the pipette tip, and the consumable storage bin 70 and the first reagent storage portion 211 extend in the X-axis direction and are arranged at an interval in the Y-axis direction. Through the above arrangement, the space of the second platform 14 in the X-axis direction and the Y-axis direction is fully utilized, which facilitates the miniaturization of the apparatus.

Specifically, the pipette tip needs to be loaded before the pipetting needle 34 transfers the first reagent and the amplification reagent, so as to avoid contamination between different reagents.

Specifically, the reagent arm 33 moves along the first guide rail 15 and the second guide rail 16, and then moves to above the consumable storage bin 70, so that the pipetting needle 34 is located in a loading position above the consumable storage bin 70, and the pipetting needle 34 moves in the Z-axis direction to load the pipette tip in the consumable storage bin 70. Then the reagent arm 33 moves along the first guide rail 15 and the second guide rail 16, so that the pipetting needle 34 moves to above the first reagent storage portion 211, and the pipetting needle 34 moves in the Z-axis direction to enable the pipette tip to suck the elution solution. Then the reagent arm 33 moves along the first guide rail 15 and the second guide rail 16, and the pipetting needle 34 moves to above the extraction reagent filling position 11 in the Z-axis direction, so as to release the elution solution in the pipette tip into the reagent tank of the corresponding first vessel. The loading process of the magnetic beads and the amplification reagent is similar to the above process and will not be elaborated herein.

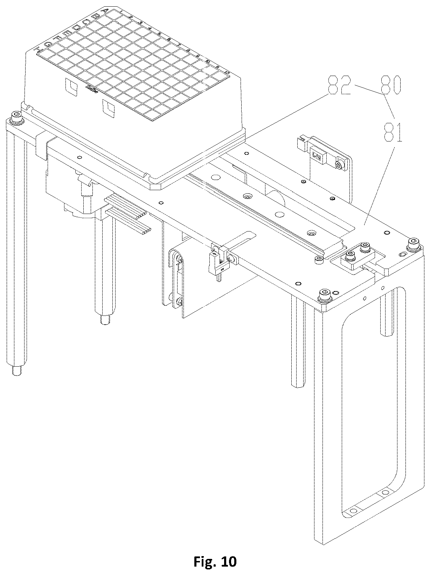

As shown in Fig. 10, the reagent preparation apparatus further includes a first vessel transfer assembly 80 arranged on the second platform 14. The first vessel transfer assembly 80 includes a transfer frame 81, a sliding plate 82, and a sliding plate driving piece. The extraction reagent filling position 11 is arranged on an upper surface of the sliding plate 82, the transfer frame 81 is arranged on the second platform 14 and extends along an X-axis, the sliding plate driving piece is arranged on the transfer frame 81, and the sliding plate driving piece is in drive connection with the sliding plate 82, so that the sliding plate 82 moves in an extension direction of the transfer frame 81. By using the first vessel transfer assembly 80, it is convenient to move the first vessel along the X-axis to cooperate with the filling of the liquid injection needle 32.

In the embodiment, because the liquid injection needle 32 only realizes the two-axis movement in the vertical plane, the sliding plate 82 needs to drive the first vessel to move along the X-axis, so that the liquid injection needle 32 is able to locate above the first vessel of the sliding plate 82.

In the embodiment, components on the second platform 14 that horizontally moves to the outside of the housing along the X-axis are removed horizontally from the same side of an instrument, including, for example: the extraction reagent storage portion 21, the second vessel loading bin 61, the consumable storage bin 70, etc., and the amplification reagent storage portion 22 partially protrudes outside of the housing 10 to facilitate the user to load the reagents, vessels and consumables on the same side of the instrument. Other mechanisms are arranged on the side, away from the above removable mechanism, of the instrument. At the same time, a vessel output mechanism is arranged on the side, away from the above movable mechanism, of the instrument, and the first vessel and the second vessel after filling output the reagent preparation apparatus from the vessel output mechanism to perform the next operation.

Specifically, an upper surface of the extraction reagent filling position 11 is lower than an upper surface of the amplification reagent filling position 12. Through the adoption of the above structure, the structure arrangement is more compact.

In the embodiment, the height of the first vessel is greater than that of the second vessel, so that the upper surface of the first vessel and the upper surface of the second vessel are of the same height through the above arrangement, thereby facilitating grabbing with the gripper 52.

The following is illustrated in combination with the use process of the reagent preparation apparatus. The user moves the first vessel loading bin 41 to the outside of the housing 10 in the X-axis direction to place the first vessel in the first vessel loading bin 41, transfers the first vessel from the first vessel loading bin 41 to the first vessel transfer bin 42 by using the horizontal transfer assembly, and moves the first vessel from the first vessel transfer bin 42 to the sliding plate 82 of the first vessel transfer assembly 80 by using the gripper 52, so that the first vessel is placed on the extraction reagent filling position 11. The liquid injection needle 32 moves to above the first vessel on the extraction reagent filling position 11 to fill the second reagent into the first vessel. The sliding plate 82 slides on the transfer frame 81, the pipetting needle 34 loaded with the pipette tip moves to the first reagent storage portion 211 to suck the first reagent, and then the pipetting needle 34 moves to above the first vessel on the extraction reagent filling position 11 to fill the first reagent into the first vessel. Thus, the preparation of the extraction reagent is realized. The user moves the second vessel loading bin 61 to the outside of the housing 10 in the X-axis direction to place the second vessel in the second vessel loading bin 61, transfers the second vessel from the second vessel loading bin 61 to the second vessel storage bin 62 by using the gripper 52, then moves the second vessel in the second vessel storage bin 62 to the amplification reagent filling position 12 by using the gripper 52, moves the first vessel from the first vessel transfer bin 42 to the premixing position 141 by using the gripper 52, loads the pipette tip by using the pipetting needle 34 and moves to the amplification reagent storage portion 22 to suck the amplification reagent, moves the pipetting needle 34 to the first vessel in the premixing position 141 to fill the amplification reagent into the first vessel and mix the amplification reagent by using the pipetting needle 34, and then sucks the mixed amplification reagent by using the pipetting needle 34 and transfers to the second vessel in the amplification reagent filling position 12, so that the preparation of the amplification reagent is realized.

Another embodiment of the invention provides a nucleic acid testing integrated machine. The nucleic acid testing integrated machine includes a reagent preparation apparatus, a to-be-tested object extraction apparatus, and an amplification apparatus. The to-be-tested object extraction apparatus is configured to extract an analyte from a sample and mix the analyte with the amplification reagent to form a determination mixture, the amplification apparatus is configured to perform analyte detection determination on the determination mixture to analyze the sample, and the reagent preparation apparatus is the above provided reagent preparation apparatus. By applying the nucleic acid testing integrated machine, when preparing the extraction reagent and the amplification reagent, the first vessel is placed on the extraction reagent filling position 11, the second vessel is placed on the amplification reagent filling position 12, the filling portion 30 which is movably arranged in the housing 10 moves to above the extraction reagent storage portion 21 to suck the extraction reagent and release the extraction reagent into the first vessel, and the filling portion 30 is able to move to above the amplification reagent storage portion 22 to suck the amplification reagent and release the amplification reagent into the second vessel. Because the preparation of the amplification reagent and the extraction reagent is completed by using the same filling portion arranged in the housing, the miniaturization of the reagent preparation apparatus is facilitated.

The reagent preparation apparatus and the amplification apparatus are respectively located on both sides of the to-be-tested object extraction apparatus, the reagent preparation apparatus, the to-be-tested object extraction apparatus and the amplification apparatus are isolated from each other, a first transfer channel is arranged between the reagent preparation apparatus and the to-be-tested object extraction apparatus, and a second transfer channel is arranged between the to-be-tested object extraction apparatus and the amplification apparatus. The first transfer channel and the second transfer channel have an on state and an off state. The nucleic acid testing machine further includes a transfer piece between the reagent preparation apparatus and the to-be-tested object extraction apparatus. The transfer piece is able to transfer the second vessel and the first vessel to the to-be-tested object extraction apparatus through the first transfer channel. Through the adoption of the above structure, when the reagent preparation apparatus or the to-be-tested object extraction apparatus works, the isolation between the reagent preparation apparatus and the to-be-tested object extraction apparatus is realized by using the first transfer channel. When the to-be-tested object extraction apparatus or the amplification apparatus works, the isolation between the to-be-tested object extraction apparatus and the amplification apparatus is realized by using the second transfer channel. The contamination between each other is avoided to improve the testing accuracy.

It is to be noted that the terms used herein is only for the purpose of describing the specific implementation modes and is not intended to limit the exemplary implementation modes of the invention. As used herein, the singular form is also intended to include the plural form unless otherwise expressly stated in the context, and it should also be understood that when the terms "contain" and/or "include" are used in the specification, they indicate the presence of features, steps, operations, devices, components and/or combinations thereof.

Unless otherwise specified, the relative arrangement, numerical expressions and numerical values of parts and steps set forth in these embodiments do not limit the scope of the invention. Also, it should be understood that, for ease of description, the dimensions of various parts shown in the accompanying drawings are not drawn to scale. Techniques, methods, and devices known to those of ordinary skill in the related fields may not be discussed in detail, but should be regarded as part of the specification under appropriate circumstances. In all examples shown and discussed herein, any specific value should be interpreted as exemplary only and not as a limitation. Thus, other examples of the exemplary embodiments may have different values. It is to be noted that: similar numbers and letters refer to similar items in the following accompanying drawings, and thus, once an item is defined in one accompanying drawing, it does not require further discussion in subsequent accompanying drawings.

In the description of the invention, it is to be understood that the orientations or positional relationships indicated by the orientation words "front, rear, upper, down, left and right", "transverse, longitudinal, vertical and horizontal", "top and bottom", etc. are based on the orientations or positional relationships shown in the accompanying drawings, and are only for the convenience of describing the invention and simplifying the description. Unless stated to the contrary, these orientation words do not indicate or imply that the apparatus or element referred to must have a specific orientation, be constructed and operated in a specific orientation, and therefore cannot be construed as limiting the scope of protection of the invention. The orientation words "inside and outside" refer to inside and outside relative to the outline of each part itself.

For ease of description, spatially relative terms, such as "over", "above", "on the surface", "upper", etc., may be used herein to describe the spatial positional relationship between a device or feature and other devices or features as shown in the figures. It should be understood that the spatially relative terms are intended to include different orientations of the device in use or operation in addition to the orientation described in the figures. For example, if the devices in the accompanying drawings are inverted, those described as "above other devices or structures" or "over other devices or structures" will then be positioned as "below other devices or structures" or "under other devices or structures". Thus, the exemplary term "above" may include both "above" and "below" orientations. The device may also be positioned in various other ways (rotated 90 degrees or at other orientations) and the spatially relative descriptions used herein are interpreted accordingly.

Furthermore, it is to be noted that the use of the words "first", "second" and the like to define parts is only for the convenience of distinguishing the corresponding parts, unless otherwise stated, the words have no special meaning, and therefore cannot be construed as limiting the scope of protection of the invention.

The above is only the preferred embodiments of the invention, and is not intended to limit the invention, and for those of ordinary skill in the art, various modifications and changes may be made to the invention within the scope of the appended claims.

## Claims

1. A reagent preparation apparatus, comprising:
a housing (10), the housing (10) being provided with an extraction reagent filling position (11) and an amplification reagent filling position (12);
a reagent storage portion (20), arranged in the housing (10), the reagent storage portion (20) comprising an extraction reagent storage portion (21) and an amplification reagent storage portion (22); and
a filling portion (30), movably arranged in the housing (10), the filling portion (30) being able to suck an extraction reagent from the extraction reagent storage portion (21) and move to above the extraction reagent filling position (11) to fill the extraction reagent into a first vessel on the extraction reagent filling position (11), and the filling portion (30) being able to suck an amplification reagent from the amplification reagent storage portion (22) and move to above the amplification reagent filling position (12) to fill the amplification reagent into a second vessel on the amplification reagent filling position (12);
wherein the housing (10) is provided with a first platform (13) and a second platform (14) located below the first platform (13), the first platform (13) and the second platform (14) are parallel to a horizontal plane, the extraction reagent storage portion (21), the amplification reagent storage portion (22), the extraction reagent filling position (11), and the amplification reagent filling position (12) are arranged on the second platform (14), and the filling portion (30) is arranged on the first platform (13);
**characterized in that**, wherein the extraction reagent storage portion (21) comprises a first reagent storage portion (211) and a second reagent storage portion;
the filling portion (30) comprises a filling assembly, wherein the filling assembly comprises a moving frame (31) and a liquid injection needle (32), wherein the moving frame (31) is movably arranged in the housing (10) in a Y-axis direction, the liquid injection needle (32) is movably arranged on the moving frame (31) in the Z-axis direction, and the liquid injection needle (32) communicates with the second reagent storage portion to fill a second reagent into the first vessel; and
the filling portion (30) further comprises a reagent arm assembly, wherein the reagent arm assembly comprises a reagent arm (33) and a pipetting needle (34), wherein the reagent arm (33) is movably arranged on the first platform (13) in the X-axis direction and the Y-axis direction, the pipetting needle (34) is movably arranged on the reagent arm (33) in the Z-axis direction, the pipetting needle (34) is able to move to above the first reagent storage portion (211) to suck a first reagent so as to fill the first reagent into the first vessel, and the pipetting needle (34) is able to move to above the amplification reagent storage portion (22) to suck the amplification reagent and move to above the amplification reagent filling position (12) to fill the amplification reagent into the second vessel.

2. The reagent preparation apparatus according to claim 1, further comprising a first vessel loading mechanism (40) configured to load the first vessel and a grabbing piece (50), wherein the first vessel loading mechanism (40) comprises a first vessel loading bin (41) and a first vessel transfer bin (42); the housing (10) is further provided with a third platform (18) located below the second platform (14) and parallel to the horizontal plane; the first vessel loading bin (41) is movably arranged on the third platform (18) and able to move to an outside of the housing (10); the first vessel transfer bin (42) is fixedly arranged on the third platform (18); a top of the first vessel transfer bin (42) is provided with a first opening (421), and the first opening (421) extends to above the second platform (14); and the grabbing piece (50) is movably arranged on the first platform (13), and the grabbing piece (50) is able to grab the first vessel through the first opening (421) and move to the extraction reagent filling position (11).

3. The reagent preparation apparatus according to claim 2, wherein the first vessel loading bin (41) and the first vessel transfer bin (42) extend in a Z-axis direction and are arranged at an interval in an X-axis direction, the housing (10) is provided with a first side opening, and the first vessel loading bin (41) is able to move to the outside of the housing (10) through the first side opening.

4. The reagent preparation apparatus according to claim 3, further comprising a second vessel loading mechanism (60) configured to load the second vessel, wherein the second vessel loading mechanism (60) comprises a second vessel loading bin (61), wherein the second vessel loading bin (61) is movably arranged in the housing (10) and able to move to the outside of the housing (10), the second vessel loading bin (61) is provided with a second opening (611), and the gripping piece (50) is able to grab the second vessel through the second opening (611).

5. The reagent preparation apparatus according to claim 4, wherein the second vessel loading mechanism (60) further comprises a second vessel storage bin (62), wherein the second vessel storage bin (62) is fixedly arranged on the second platform (14) and extends in the Z-axis direction, and the second vessel loading bin (61) is movably arranged on the second platform (14) in the X-axis direction and extends in the Z-axis direction; the housing (10) is further provided with a second side opening located above the first side opening; the second vessel loading bin (61) is able to move to the outside of the housing through the second side opening; a top of the second vessel storage bin (62) is provided with a third opening (621); and the grabbing piece (50) is able to transfer the second vessel from the second vessel loading bin (61) to the second vessel storage bin (62), and the grabbing piece (50) is able to grab the second vessel through the third opening (621) and move to the amplification reagent filling position (12).

6. The reagent preparation apparatus according to claim 5, wherein the first reagent storage portion (211) is arranged on the second platform (14), the second reagent storage portion is arranged outside of the housing (10), the first reagent storage portion (211) and the second vessel loading bin (61) are arranged at an interval in the X-axis direction, and the first reagent storage portion (211) is able to move to the outside of the housing (10) with the second vessel loading bin (61).

7. The reagent preparation apparatus according to claim 2, wherein the second platform (14) is further provided with a premixing position (141) configured to place the first vessel, and the grabbing piece (50) is able to grab the first vessel through the first opening (421) and move to the premixing position (141).

8. The reagent preparation apparatus according to claim 1, wherein the first platform (13) is provided with a first guide rail (15) and a second guide rail (16), the first guide rail (15) extends in the Y-axis direction, the second guide rail (16) extends in the X-axis direction, the first guide rail (15) is movably arranged below the second guide rail (16) in an extension direction of the second guide rail (16), and the reagent arm (33) is movably arranged on the first guide rail (15) in an extension direction of the first guide rail (15).

9. The reagent preparation apparatus according to claim 8, wherein the first platform (13) is further provided with a third guide rail (17) extending in the Y-axis direction, and the third guide rail (17) is movably arranged below the second guide rail (16) in the extension direction of the second guide rail (16); and the grabbing piece (50) comprises a grabbing frame (51) and a gripper (52), wherein the grabbing frame (51) is movably arranged on the third guide rail (17) in an extension direction of the third guide rail (17), and the gripper (52) is movably arranged on the grabbing frame (51) in the Z-axis direction.

10. The reagent preparation apparatus according to claim 1, further comprising a consumable storage bin (70) arranged on the second platform (14) and configured to carry a magnetic rod sleeve and a pipette tip, wherein the pipetting needle (34) is able to move to above the consumable storage bin (70) to load the magnetic rod sleeve and the pipette tip, and the consumable storage bin (70) and the first reagent storage portion (211) extend in the X-axis direction and are arranged at an interval in the Y-axis direction.

11. The reagent preparation apparatus according to claim 2, further comprising a first vessel transfer assembly (80) arranged on the second platform (14), wherein the first vessel transfer assembly (80) comprises a transfer frame (81), a sliding plate (82), and a sliding plate driving piece, wherein the extraction reagent filling position (11) is arranged on an upper surface of the sliding plate (82), the transfer frame (81) is arranged on the second platform (14) and extends in the X-axis direction, the sliding plate driving piece is arranged on the transfer frame (81), and the sliding plate driving piece is in drive connection with the sliding plate (82), so that the sliding plate (82) moves in an extension direction of the transfer frame (81).

12. The reagent preparation apparatus according to claim 1, wherein an upper surface of the extraction reagent filling position (11) is lower than an upper surface of the amplification reagent filling position (12).

13. A nucleic acid testing integrated machine, comprising a reagent preparation apparatus, a to-be-tested object extraction apparatus, and an amplification apparatus, wherein the to-be-tested object extraction apparatus is configured to extract an analyte from a sample and mix the analyte with the amplification reagent to form a determination mixture, the amplification apparatus is configured to perform analyte detection determination on the determination mixture to analyze the sample, and the reagent preparation apparatus is the reagent preparation apparatus according to any one of claims 1-12.

## Patentansprüche

1. Vorrichtung zur Herstellung von Reagenzien, umfassend:
ein Gehäuse (10), wobei das Gehäuse (10) mit einer Extraktionsreagenzfüllposition (11) und einer Amplifikationsreagenzfüllposition (12) bereitgestellt ist;
einen Reagenzspeicherabschnitt (20), der in dem Gehäuse (10) angeordnet ist, wobei der Reagenzspeicherabschnitt (20) einen Extraktionsreagenzspeicherabschnitt (21) und einen Amplifikationsreagenzspeicherabschnitt (22) umfasst; und
einen Füllabschnitt (30), der beweglich in dem Gehäuse (10) angeordnet ist, wobei der Füllabschnitt (30) in der Lage ist, ein Extraktionsreagenz aus dem Extraktionsreagenzspeicherabschnitt (21) anzusaugen und sich über die Extraktionsreagenz-Füllposition (11) zu bewegen, um das Extraktionsreagenz in ein erstes Gefäß an der Extraktionsreagenzfüllposition (11) zu füllen, und wobei der Füllabschnitt (30) in der Lage ist, ein Amplifikationsreagenz aus dem Amplifikationsreagenzspeicherabschnitt (22) anzusaugen und sich vorstehend über die Amplifikationsreagenzfüllposition (12) zu bewegen, um das Amplifikationsreagenz in ein zweites Gefäß an der Amplifikationsreagenzfüllposition (12) zu füllen;
wobei das Gehäuse (10) mit einer ersten Plattform (13) und einer zweiten Plattform (14) bereitgestellt ist, die sich unterhalb der ersten Plattform (13) befindet, wobei die erste Plattform (13) und die zweite Plattform (14) parallel zu einer horizontalen Ebene sind, wobei der Extraktionsreagenz-Speicherabschnitt (21), der Amplifikationsreagenz-Speicherabschnitt (22), die Extraktionsreagenz-Füllposition (11) und die Amplifikationsreagenz-Füllposition (12) auf der zweiten Plattform (14) angeordnet sind und der Füllabschnitt (30) auf der ersten Plattform (13) angeordnet ist;
**dadurch gekennzeichnet, dass** der Extraktionsreagenzspeicherabschnitt (21) einen ersten Reagenzspeicherabschnitt (211) und einen zweiten Reagenzspeicherabschnitt umfasst;
der Füllabschnitt (30) eine Füllbaugruppe umfasst, wobei die Füllbaugruppe einen beweglichen Rahmen (31) und eine Flüssigkeitsinjektionsnadel (32) umfasst, wobei der bewegliche Rahmen (31) in einer Y-Achsenrichtung beweglich in dem Gehäuse (10) angeordnet ist, die Flüssigkeitsinjektionsnadel (32) in einer Z-Achsenrichtung beweglich auf dem beweglichen Rahmen (31) angeordnet ist, und die Flüssigkeitsinjektionsnadel (32) mit dem zweiten Reagenzspeicherabschnitt in Verbindung steht, um ein zweites Reagenz in das erste Gefäß zu füllen; und
der Füllabschnitt (30) weiter eine Reagenzarmbaugruppe umfasst, wobei die Reagenzarmbaugruppe einen Reagenzarm (33) und eine Pipettiernadel (34) umfasst, wobei der Reagenzarm (33) auf der ersten Plattform (13) in X-Achsenrichtung und Y-Achsenrichtung beweglich angeordnet ist, die Pipettiernadel (34) auf dem Reagenzarm (33) in Z-Achsenrichtung beweglich angeordnet ist, die Pipettiernadel (34) vorstehend über den ersten Reagenzspeicherabschnitt (211) bewegen, um ein erstes Reagenz anzusaugen, um das erste Reagenz in das erste Gefäß zu füllen, und die Pipettiernadel (34) sich über den Amplifikationsreagenz-Speicherabschnitt (22) bewegen kann, um das Amplifikationsreagenz anzusaugen, und sich über die Amplifikationsreagenz-Füllposition (12) bewegen kann, um das Amplifikationsreagenz in das zweite Gefäß zu füllen.

2. Vorrichtung zur Herstellung von Reagenzien nach Anspruch 1, die weiter einen ersten Gefäßlademechanismus (40) umfasst, der zum Laden des ersten Gefäßes und eines Greifstücks (50) konfiguriert ist, wobei der erste Gefäßlademechanismus (40) einen ersten Gefäßladebehälter (41) und einen ersten Gefäßtransferbehälter (42) umfasst; das Gehäuse (10) ist weiter mit einer dritten Plattform (18) bereitgestellt, die sich unterhalb der zweiten Plattform (14) und parallel zur horizontalen Ebene befindet; der erste Gefäßladebehälter (41) ist beweglich auf der dritten Plattform (18) angeordnet und kann sich zu einer Außenseite des Gehäuses (10) bewegen; der erste Gefäßtransferbehälter (42) fest auf der dritten Plattform (18) angeordnet ist; eine Oberseite des ersten Gefäßtransferbehälters (42) mit einer ersten Öffnung (421) bereitgestellt ist und sich die erste Öffnung (421) bis vorstehend über die zweite Plattform (14) erstreckt; und das Greifstück (50) ist beweglich auf der ersten Plattform (13) angeordnet, und das Greifstück (50) kann das erste Gefäß durch die erste Öffnung (421) greifen und sich zur Extraktionsreagenzfüllposition (11) bewegen.

3. Vorrichtung zur Herstellung von Reagenzien nach Anspruch 2, wobei sich der erste Gefäßladebehälter (41) und der erste Gefäßtransferbehälter (42) in Z-Achsenrichtung erstrecken und in einem Abstand in X-Achsenrichtung angeordnet sind, das Gehäuse (10) mit einer ersten Seitenöffnung bereitgestellt ist und der erste Gefäßladebehälter (41) durch die erste Seitenöffnung zur Außenseite des Gehäuses (10) bewegt werden kann.

4. Vorrichtung zur Herstellung von Reagenzien nach Anspruch 3, die weiter einen zweiten Gefäßlademechanismus (60) umfasst, der zum Laden des zweiten Gefäßes konfiguriert ist, wobei der zweite Gefäßlademechanismus (60) einen zweiten Gefäßladebehälter (61) umfasst, wobei der zweite Gefäßladebehälter (61) beweglich in dem Gehäuse (10) angeordnet ist und sich zur Außenseite des Gehäuses (10) bewegen kann, der zweite Gefäßladebehälter (61) mit einer zweiten Öffnung (611) bereitgestellt ist und das Greifstück (50) in der Lage ist, das zweite Gefäß durch die zweite Öffnung (611) einzuklemmen.

5. Vorrichtung zur Herstellung von Reagenzien nach Anspruch 4, wobei der zweite Gefäßlademechanismus (60) weiterhin einen zweiten Gefäßspeicherbehälter (62) umfasst, wobei der zweite Gefäßspeicherbehälter (62) fest auf der zweiten Plattform (14) angeordnet ist und sich in der Z-Achsenrichtung erstreckt, und der zweite Gefäßladebehälter (61) beweglich auf der zweiten Plattform (14) in der X-Achsenrichtung angeordnet ist und sich in der Z-Achsen-Richtung erstreckt; das Gehäuse (10) ist weiterhin mit einer zweiten seitlichen Öffnung bereitgestellt, die sich über der ersten seitlichen Öffnung befindet; der zweite Gefäßladebehälter (61) ist in der Lage, sich durch die zweite Seitenöffnung zur Außenseite des Gehäuses zu bewegen; eine Oberseite des zweiten Gefäßspeicherbehälters (62) ist mit einer dritten Öffnung (621) bereitgestellt; und das Greifteil (50) ist in der Lage, das zweite Gefäß vom zweiten Gefäßladebehälter (61) zum zweiten Gefäßspeicherbehälter (62) zu transferieren, und das Greifteil (50) ist in der Lage, das zweite Gefäß durch die dritte Öffnung (621) zu ergreifen und sich zur Füllposition (12) für das Verstärkungsreagenz zu bewegen.

6. Vorrichtung zur Herstellung von Reagenzien nach Anspruch 5, wobei der erste Abschnitt (211) zur Aufbewahrung von Reagenzien auf der zweiten Plattform (14) angeordnet ist, der zweite Abschnitt zur Aufbewahrung von Reagenzien außerhalb des Gehäuses (10) angeordnet ist, der erste Abschnitt (211) zur Aufbewahrung von Reagenzien und der zweite Gefäßladebehälter (61) in einem Abstand in Richtung der X-Achse angeordnet sind, und der erste Abschnitt (211) zur Aufbewahrung von Reagenzien in der Lage ist, sich mit dem zweiten Gefäßladebehälter (61) zur Außenseite des Gehäuses (10) zu bewegen.

7. Vorrichtung zur Herstellung von Reagenzien nach Anspruch 2, wobei die zweite Plattform (14) weiter mit einer Vormischposition (141) bereitgestellt ist, die zum Platzieren des ersten Gefäßes konfiguriert ist, und das Greifelement (50) in der Lage ist, das erste Gefäß durch die erste Öffnung (421) zu greifen und sich zur Vormischposition (141) zu bewegen.

8. Vorrichtung zur Herstellung von Reagenzien nach Anspruch 1, wobei die erste Plattform (13) mit einer ersten Führungsschiene (15) und einer zweiten Führungsschiene (16) bereitgestellt ist, wobei sich die erste Führungsschiene (15) in Y-Achsenrichtung erstreckt, die zweite Führungsschiene (16) sich in X-Achsenrichtung erstreckt, die erste Führungsschiene (15) unterhalb der zweiten Führungsschiene (16) in einer Ausdehnungsrichtung der zweiten Führungsschiene (16) beweglich angeordnet ist und der Reagenzarm (33) auf der ersten Führungsschiene (15) in einer Ausdehnungsrichtung der ersten Führungsschiene (15) beweglich angeordnet ist.

9. Vorrichtung zur Herstellung von Reagenzien nach Anspruch 8, wobei die erste Plattform (13) weiter mit einer dritten Führungsschiene (17) bereitgestellt ist, die sich in Y-Richtung erstreckt, und die dritte Führungsschiene (17) unterhalb der zweiten Führungsschiene (16) in Verlängerungsrichtung der zweiten Führungsschiene (16) beweglich angeordnet ist; und das Greifstück (50) einen Greifrahmen (51) und einen Greifer (52) umfasst, wobei der Greifrahmen (51) auf der dritten Führungsschiene (17) in einer Ausfahrrichtung der dritten Führungsschiene (17) beweglich angeordnet ist und der Greifer (52) auf dem Greifrahmen (51) in Z-Achsenrichtung beweglich angeordnet ist.

10. Vorrichtung zur Herstellung von Reagenzien nach Anspruch 1, die weiter einen Verbrauchsmaterialbehälter (70) umfasst, der auf der zweiten Plattform (14) angeordnet und so konfiguriert ist, dass er eine Magnetstangenhülse und eine Pipettenspitze aufnehmen kann, wobei die Pipettiernadel (34) sich über den Verbrauchsmaterialbehälter (70) bewegen kann, um die Magnetstangenhülse und die Pipettenspitze aufzunehmen, und das Verbrauchsmaterial-Bin (70) und der erste Reagenz-Abschnitt (211) sich in X-Achsenrichtung erstrecken und in Y-Achsenrichtung in einem Abstand voneinander angeordnet sind.

11. Vorrichtung zur Herstellung von Reagenzien nach Anspruch 2, die weiter eine erste Behältertransferbaugruppe (80) umfasst, die auf der zweiten Plattform (14) angeordnet ist, wobei die erste Behältertransferbaugruppe (80) einen Transferrahmen (81), eine Gleitplatte (82) und ein Gleitplattenantriebsstück umfasst, wobei die Extraktionsreagenzfüllposition (11) auf einer Oberseite der Gleitplatte (82) angeordnet ist, der Transferrahmen (81) auf der zweiten Plattform (14) angeordnet ist und sich in X-Achsenrichtung erstreckt, das Schiebeplattenantriebsstück auf dem Transferrahmen (81) angeordnet ist und das Schiebeplattenantriebsstück mit der Schiebeplatte (82) verbunden ist, so dass sich die Schiebeplatte (82) in einer Ausfahrrichtung des Transferrahmens (81) bewegt.

12. Vorrichtung zur Herstellung von Reagenzien nach Anspruch 1, wobei eine Oberseite der Extraktionsreagenzfüllposition (11) niedriger ist als eine Oberseite der Amplifikationsreagenzfüllposition (12).

13. Integrierte Maschine zur Nukleinsäureuntersuchung, umfassend eine Vorrichtung zur Herstellung von Reagenzien, eine Extraktionsvorrichtung für zu untersuchende Objekte und eine Amplifikationsvorrichtung, wobei die Extraktionsvorrichtung für zu untersuchende Objekte so konfiguriert ist, dass sie einen Analyten aus einer Probe extrahiert und den Analyten mit dem Amplifikationsreagenz mischt, um eine Bestimmungsmischung zu bilden, wobei die Amplifikationsvorrichtung so konfiguriert ist, dass sie eine Analytdetektionsbestimmung an der Mischung durchführt, um die Probe zu analysieren, und wobei die Vorrichtung zur Herstellung von Reagenzien die Vorrichtung zur Herstellung von Reagenzien nach einem der Ansprüche 1 bis 12 ist.

## Revendications

1. Appareil de préparation de réactif comprenant :
un boîtier (10), le boîtier (10) étant pourvu d'une position de remplissage (11) de réactif d'extraction et d'une position de remplissage (12) de réactif d'amplification ;
une partie de stockage (20) de réactif, disposée dans le boîtier (10), la partie de stockage (20) de réactif comprenant une partie de stockage (21) de réactif d'extraction et une partie de stockage (22) de réactif d'amplification ; et
une partie de remplissage (30), disposée de manière mobile dans le boîtier (10), la partie de remplissage (30) pouvant aspirer un réactif d'extraction à partir de la partie de stockage (21) de réactif d'extraction et se déplacer au-dessus de la position de remplissage (11) de réactif d'extraction pour remplir le réactif d'extraction dans un premier récipient sur la position de remplissage (11) de réactif d'extraction, et la partie de remplissage (30) pouvant aspirer un réactif d'amplification à partir de la partie de stockage (22) de réactif d'amplification et se déplacer au-dessus de la position de remplissage (12) de réactif d'amplification pour remplir le réactif d'amplification dans un second récipient sur la position de remplissage (12) de réactif d'amplification ;
le boîtier (10) étant pourvu d'une première plateforme (13) et d'une deuxième plateforme (14) située sous la première plateforme (13), la première plateforme (13) et la deuxième plateforme (14) étant parallèles à un plan horizontal, la partie de stockage (21) de réactif d'extraction, la partie de stockage (22) de réactif d'amplification, la position de remplissage (11) de réactif d'extraction et la position de remplissage (12) de réactif d'amplification étant disposées sur la deuxième plateforme (14), et la partie de remplissage (30) étant disposée sur la première plateforme (13) ;
**caractérisé en ce que** la partie de stockage (21) de réactif d'extraction comprend une première partie de stockage (211) de réactif et une seconde partie de stockage de réactif ;
la partie de remplissage (30) comprend un ensemble de remplissage, l'ensemble de remplissage comprenant un cadre mobile (31) et une aiguille d'injection de liquide (32), le cadre mobile (31) étant disposé de manière mobile dans le boîtier (10) dans une direction d'axe Y, l'aiguille d'injection de liquide (32) étant disposée de manière mobile sur le cadre mobile (31) dans la direction d'axe Z, et l'aiguille d'injection de liquide (32) communiquant avec la seconde partie de stockage de réactif pour remplir un second réactif dans le premier récipient ; et
la partie de remplissage (30) comprend en outre un ensemble bras de réactif, l'ensemble bras de réactif comprenant un bras de réactif (33) et une aiguille de pipetage (34), le bras de réactif (33) étant disposé de manière mobile sur la première plateforme (13) dans la direction de l'axe X et la direction de l'axe Y, l'aiguille de pipetage (34) étant disposée de manière mobile sur le bras de réactif (33) dans la direction de l'axe Z, l'aiguille de pipetage (34) pouvant se déplacer au-dessus de la première partie de stockage (211) de réactif pour aspirer un premier réactif afin de remplir le premier réactif dans le premier récipient, et l'aiguille de pipetage (34) pouvant se déplacer au-dessus de la partie de stockage (22) de réactif d'amplification pour aspirer le réactif d'amplification et se déplacer au-dessus de la position de remplissage (12) de réactif d'amplification pour remplir le réactif d'amplification dans le second récipient.

2. Appareil de préparation de réactif selon la revendication 1, comprenant en outre un premier mécanisme de chargement (40) de récipient configuré pour charger le premier récipient et une pièce de préhension (50), le premier mécanisme de chargement (40) de récipient comprenant un premier bac de chargement (41) de récipient et un premier bac de transfert (42) de récipient ; le boîtier (10) étant en outre pourvu d'une troisième plateforme (18) située sous la deuxième plateforme (14) et parallèle au plan horizontal ; le premier bac de chargement (41) de récipient étant disposé de manière mobile sur la troisième plateforme (18) et pouvant se déplacer vers l'extérieur du boîtier (10) ; le premier bac de transfert (42) de récipient étant disposé de manière fixe sur la troisième plateforme (18) ; une partie supérieure du premier bac de transfert (42) de récipient étant pourvue d'une première ouverture (421), et la première ouverture (421) s'étendant jusqu'au-dessus de la deuxième plateforme (14) ; et la pièce de préhension (50) étant disposée de manière mobile sur la première plateforme (13), et la pièce de préhension (50) étant capable de saisir le premier récipient à travers la première ouverture (421) et de se déplacer jusqu'à la position de remplissage (11) de réactif d'extraction.

3. Appareil de préparation de réactif selon la revendication 2, le premier bac de chargement (41) de récipient et le premier bac de transfert (42) de récipient s'étendant dans la direction de l'axe Z et étant disposés à un certain intervalle dans la direction de l'axe X, le boîtier (10) étant pourvu d'une première ouverture latérale, et le premier bac de chargement (41) de récipient pouvant se déplacer vers l'extérieur du boîtier (10) à travers la première ouverture latérale.

4. Appareil de préparation de réactif selon la revendication 3, comprenant en outre un second mécanisme de chargement (60) de récipient configuré pour charger le second récipient, le second mécanisme de chargement (60) de récipient comprenant un second bac de chargement (61) de récipient, le second bac de chargement (61) de récipient étant disposé de manière mobile dans le boîtier (10) et pouvant se déplacer vers l'extérieur du boîtier (10), le second bac de chargement (61) de récipient étant pourvu d'une deuxième ouverture (611), et la pièce de préhension (50) étant capable de saisir le second récipient à travers la deuxième ouverture (611).

5. Appareil de préparation de réactif selon la revendication 4, le second mécanisme de chargement (60) de récipient comprenant en outre un second bac de stockage (62) de récipient, le second bac de stockage (62) de récipient étant disposé de manière fixe sur la deuxième plateforme (14) et s'étendant dans la direction de l'axe Z, et le second bac de chargement (61) de récipient étant disposé de manière mobile sur la deuxième plateforme (14) dans la direction de l'axe X et s'étendant dans la direction de l'axe Z ; le boîtier (10) étant en outre pourvu d'une deuxième ouverture latérale située au-dessus de la première ouverture latérale ; le second bac de chargement (61) de récipient pouvant se déplacer vers l'extérieur du boîtier à travers la deuxième ouverture latérale ; une partie supérieure du second bac de stockage (62) de récipient étant pourvue d'une troisième ouverture (621) ; et la pièce de préhension (50) pouvant transférer le second récipient du second bac de chargement (61) de récipient au second bac de stockage (62) de récipient, et la pièce de préhension (50) pouvant saisir le second récipient à travers la troisième ouverture (621) et se déplacer vers la position de remplissage (12) de réactif d'amplification.

6. Appareil de préparation de réactif selon la revendication 5, la première partie de stockage (211) de réactif étant disposée sur la deuxième plateforme (14), la deuxième partie de stockage de réactif étant disposée à l'extérieur du boîtier (10), la première partie de stockage (211) de réactif et le second bac de chargement (61) de récipient étant disposés à un certain intervalle dans la direction de l'axe X, et la première partie de stockage (211) de réactif pouvant se déplacer vers l'extérieur du boîtier (10) avec le second bac de chargement (61) de récipient.

7. Appareil de préparation de réactif selon la revendication 2, la deuxième plateforme (14) étant en outre pourvue d'une position de prémélange (141) configurée pour placer le premier récipient, et la pièce de préhension (50) étant capable de saisir le premier récipient à travers la première ouverture (421) et de se déplacer vers la position de prémélange (141).

8. Appareil de préparation de réactif selon la revendication 1, la première plateforme (13) étant pourvue d'un premier rail de guidage (15) et d'un deuxième rail de guidage (16), le premier rail de guidage (15) s'étendant dans la direction de l'axe Y, le deuxième rail de guidage (16) s'étendant dans la direction de l'axe X, le premier rail de guidage (15) étant disposé de manière mobile sous le deuxième rail de guidage (16) dans une direction d'extension du deuxième rail de guidage (16), et le bras de réactif (33) étant disposé de manière mobile sur le premier rail de guidage (15) dans une direction d'extension du premier rail de guidage (15).

9. Appareil de préparation de réactif selon la revendication 8, la première plateforme (13) étant en outre pourvue d'un troisième rail de guidage (17) s'étendant dans la direction de l'axe Y, et le troisième rail de guidage (17) étant disposé de manière mobile sous le deuxième rail de guidage (16) dans la direction d'extension du deuxième rail de guidage (16) ; et la pièce de préhension (50) comprenant un cadre de préhension (51) et un préhenseur (52), le cadre de préhension (51) étant disposé de manière mobile sur le troisième rail de guidage (17) dans une direction d'extension du troisième rail de guidage (17), et le préhenseur (52) étant disposé de manière mobile sur le cadre de préhension (51) dans la direction de l'axe Z.

10. Appareil de préparation de réactif selon la revendication 1, comprenant en outre un bac de stockage (70) de consommables disposé sur la deuxième plateforme (14) et configuré pour transporter un manchon de tige magnétique et une pointe de pipette, l'aiguille de pipetage (34) pouvant se déplacer au-dessus du bac de stockage (70) de consommables pour charger le manchon de tige magnétique et la pointe de pipette, et le bac de stockage (70) de consommables et la première partie de stockage (211) de réactif s'étendant dans la direction de l'axe X et étant disposés à un certain intervalle dans la direction de l'axe Y.

11. Appareil de préparation de réactif selon la revendication 2, comprenant en outre un premier ensemble de transfert (80) de récipient disposé sur la deuxième plateforme (14), le premier ensemble de transfert (80) de récipient comprenant un cadre de transfert (81), une plaque coulissante (82), et une pièce d'entraînement de plaque coulissante, la position de remplissage (11) de réactif d'extraction étant disposée sur une surface supérieure de la plaque coulissante (82), le cadre de transfert (81) étant disposé sur la deuxième plateforme (14) et s'étendant dans la direction de l'axe X, la pièce d'entraînement de plaque coulissante étant disposée sur le cadre de transfert (81), et la pièce d'entraînement de plaque coulissante étant en liaison d'entraînement avec la plaque coulissante (82), de sorte que la plaque coulissante (82) se déplace dans une direction d'extension du cadre de transfert (81).

12. Appareil de préparation de réactif selon la revendication 1, une surface supérieure de la position de remplissage (11) de réactif d'extraction étant inférieure à une surface supérieure de la position de remplissage (12) de réactif d'amplification.

13. Machine intégrée de test d'acide nucléique, comprenant un appareil de préparation de réactif, un appareil d'extraction de l'objet à tester et un appareil d'amplification, l'appareil d'extraction de l'objet à tester étant configuré pour extraire un analyte d'un échantillon et mélanger l'analyte avec le réactif d'amplification pour former un mélange de détermination, l'appareil d'amplification étant configuré pour effectuer une détermination de détection de l'analyte sur le mélange de détermination pour analyser l'échantillon, et l'appareil de préparation de réactif étant l'appareil de préparation de réactif selon l'une quelconque des revendications 1-12.
